# FASCICULE DE BREVET EUROPEEN

(11) **EP 0 314 583 B1**
(45) Date de publication et mention de la délivrance du brevet: **19.05.1993**
(21) Numéro de dépôt: 88420351.4
(22) Date de dépôt: 14.10.1988
(51) Int. Cl.: C07C 37/60, C07C 39/08

(54) **Procédé d'hydroxylation de phénols et d'éthers de phénols**
Verfahren zur Hydroxylierung von Phenolen und Ätherphenolen
Process for the hydroxylation of phenols and phenol-ethers

(30) Priorité: 29.10.1987 FR 8715248
(43) Date de publication de la demande: 03.05.1989
(73) Titulaire: RHONE-POULENC CHIMIE, 92408 Courbevoie Cédex (FR)
(72) Inventeur: Costantini, Michel, F-69003 - Lyon (FR); Popa, Jean-Michel, F-93700 - Drancy (FR); Gubelmann, Michel, F-69006 - Lyon (FR)
(74) Mandataire: Dutruc-Rosset, Marie-Claude

(56) Documents cités:
- EP-A- 0 095 304
- EP-A- 0 107 385
- GB-A- 2 116 974
- US-A- 3 580 956
- US-A- 4 578 521

## Description

La présente invention concerne un procédé d'hydroxylation de phénols ou d'éthers de phénols par le peroxyde d'hydrogène.

L'hydroxylation du phénol ou de phénols substitués, par le peroxyde d'hydrogène, pour préparer des diphénols est une réaction connue.

Le brevet français n^{o} 69-45467 publié sous le n^{o} 2 071 464 a décrit un procédé dans lequel la réaction est catalysée par un acide fort, tel que par exemple l'acide perchlorique ou l'acide sulfurique.

Le brevet allemand n^{o} 2 410 742 a décrit un procédé semblable au précédent, dans lequel le peroxyde d'hydrogène est mis en oeuvre sous forme de solution organique pratiquement anhydre.

Ces deux procédés sont très intéressants et le premier procédé est mis en oeuvre industriellement.

Cependant depuis quelques années, on cherche à catalyser la réaction d'hydroxylation à l'aide de solides non dissous dans le milieu, afin de simplifier leur séparation du milieu réactionnel et leur éventuel recyclage et éviter les sous-produits salins, qui se forment le plus souvent lors de l'élimination des catalyseurs acides dissous.

Il est connu d'effectuer l'hydroxylation du phénol par le peroxyde d'hydrogène, en présence d'une zéolithe de type ZSM-5 (US-A 4 578 521) ou d'une zéolithe naturelle telle que la faujasite ou la mordénite (US-A 3 580 956).

La demande de brevet français n^{o} 81-17023 (publiée sous le n^{o} 2 489 816) préconise l'emploi de silicalite de titane comme catalyseur hétérogène de l'hydroxylation de composés aromatiques par le peroxyde d'hydrogène.

En fait, cette catalyse présente de grandes difficultés de reproductibilité. En outre la fine taille des particules de catalyseur utilisées rend leur séparation du milieu réactionnel très difficile et leur recyclage problématique, alors que dans un procédé industriel, il est indispensable de pouvoir recycler un catalyseur coûteux.

Pour remédier à ce problème de la séparation du catalyseur, il a été proposé, dans la demande de brevet européen publiée sous le n^{o} 200 260, d'utiliser des agglomérats de ces fines particules de silicalite de titane.

Il s'avère cependant que l'on recherche encore une catalyse hétérogène de la réaction d'hydroxylation des phénols ou éthers de phénols par le peroxyde d'hydrogène, qui puisse être utilisée de manière industrielle dans des conditions économiquement acceptables. C'est précisément l'objet de la présente invention.

L'invention consiste donc en un procédé d'hydroxylation de phénols ou éthers de phénols de formule générale (I) : 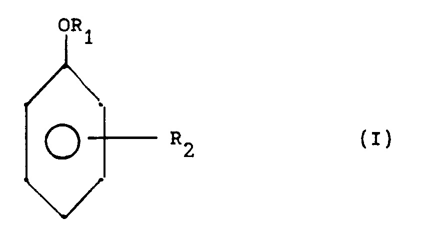
dans laquelle :
- R₁ représente un atome d'hydrogène, un groupement méthyle, un groupement éthyle ou un groupement phényle,
- R₂ représente un atome d'hydrogène, un radical alkyle ayant 1 à 4 atomes de carbone, un radical alkoxy ayant 1 à 4 atomes de carbone, un radical phényle ou cyclohexyle ;
par réaction avec le peroxyde d'hydrogène, caractérisé en ce que la réaction est conduite en présence d'une quantité efficace d'un catalyseur constitué d'au moins une argile acide.

Par argiles acides que l'on utilise dans le cadre du procédé de l'invention, on entend les argiles qui présentent en suspension dans l'eau un pH inférieur à 7.

De préférence ce pH, mesuré dans des conditions décrites ci-après, est compris entre 2 et 6.

La mesure du pH des argiles acides peut se faire de la manière suivante :
- on prépare une suspension de l'argile dans l'eau par pesée de 2 grammes de l'argile broyée et addition de 100 cm3 d'eau permutée ;
- on agite pendant 10 minutes environ à l'aide d'un agitateur magnétique ;
- on mesure le pH à l'aide d'une électrode double pendant que la suspension est maintenue sous agitation.

Les argiles utilisables dans le procédé de la présente invention sont choisies de préférence parmi les argiles naturelles présentant une structure dite "TOT" ou tetraèdre-octaèdre-tétraèdre.

Les argiles sont divisées en 3 classes :
- les smectites,
- les vermiculites,
- les micas.

Les argiles TOT se présentent sous forme de feuillets élémentaires comprenant deux couches de tétraèdres d'atomes d'oxygène dans lesquels sont inclus les atomes de silicium séparées par une couche d'octaèdres d'atomes d'oxygène dans lesquels est inclus le métal M de type (MO₄OH₂) où M est un cation di ou trivalent.

Lorsque tous les tétraèdres sont occupés par des ions Si⁴⁺ la neutralité électrique du feuillet peut être assurée de deux façons, selon la charge du cation octaédrique :
- s'il est divalent (Mg²⁺, Fe²⁺, ...), toutes les cavités octaédriques sont occupées. Le feuillet est alors dit trioctaédrique ;
- s'il est trivalent (Al³⁺, Fe³⁺, ...), deux cavités octaédriques sur trois sont occupées et le feuillet est dioctaédrique.

Mais de nombreuses substitutions sont possibles, aussi bien dans la couche tétraédrique que dans la couche octaédrique. Celles-ci peuvent entraîner un déficit de charge du feuillet et la neutralité du cristal est alors réalisée par insertion de cations compensateurs entre les feuillets.

On préfère utiliser les smectites.

Les smectites sont classifiées selon la nature du métal M (aluminium, magnésium, fer, lithium) et la nature du cation compensateur (sodium, potassium, calcium).

On peut ainsi citer parmi la classe des smectites :
- les montmorillonites de formule générale Si₄(Al_{2-y}-Mg_{y})O₁₀OH₂,M⁺_{Y}
- les beidellites de formule (Si₄₋ₓAlₓ)Al₂O₁₀OH₂, M⁺ₓ
- les nontronites de formule (Si₄₋ₓAlₓ)Fe₂O₁₀OH₂,M⁺ₓ
- les hectorites de formule Si₄(Mg_{3-y}-Li_{y})O₁₀OH₂,M⁺_{y}
- les stévensites de formule Si₄(Mg_{3-y})O₁₀OH₂,M⁺_{2y}
- les saponites de formule (Si₄₋ₓAlₓ)Mg₃O₁₀OH₂,M⁺ₓ.

On préfère encore tout particulièrement dans le cadre de la présente invention utiliser les montmorillonites.

On peut utiliser des argiles commerciales déjà acides telles que notamment les argiles suivantes :
. KSF vendue par Süd-Chemie (Münich),
. TONSIL OPTIMUM FF vendue par Süd-Chemie,
. K 10 vendue par Süd-Chemie.

L'argile KSF a une surface de 20 à 40 m²/g et une densité de 800 à 850 g/l.

L'argile K 10 a une surface de 220 à 270 m²/g et une densité de 300 à 370 g/l.

L'argile TONSIL OPTIMUM FF a une surface spécifique d'environ 270 m²/g.

On peut également traiter les argiles commerciales, déjà acides ou non par une solution aqueuse d'un acide. La concentration de la solution aqueuse en acide est variable, cependant elle devra avoir un pH supérieur ou égal à 2 de façon à ne pas détruire l'argile et elle devra contenir une quantité d'ion H⁺ exprimé en milliéquivalents correspondant à au moins la capacité d'échange de l'argile. On définit la capacité d'échange d'une argile comme le nombre de cations monovalents, exprimé en milliéquivalents que peuvent échanger 100 g d'échantillon.

Cette capacité d'échange (ou charge par demi-maille) varie pour les smectites entre 0,2 et 0,6 et pour les vermiculites entre 0,6 et 0,9. Il est préférable donc dans le cadre de la présente invention d'utiliser une solution d'acide contenant autant d'équivalents H⁺ qu'il y aura de cations à échanger dans l'argile donc contenant au moins 0,6 et de préférence au moins 1 milliéquivalent acide pour 100 g d'argile.

Ce traitement de l'argile est très facile à mettre en oeuvre. Parmi les acides utilisés on peut citer l'acide chlorhydrique, l'acide sulfurique, l'acide nitrique,l'acide trifluorométhanesulfonique et l'acide phosphorique. Les acides organiques pourraient aussi être utilisés, mais ne présentent aucun avantage par rapport aux acides minéraux et présentent surtout l'inconvénient d'être plus onéreux.

On peut éventuellement, pour améliorer la surface d'échange de l'argile, la traiter ensuite avec un alcool tel que le méthanol, puis la sécher.

Les phénols et éthers de phénols qui sont utilisés de préférence dans le procédé de l'invention sont les composés de formule (I) dans laquelle R₁ représente un atome d'hydrogène, un groupement méthyle ou un groupement éthyle, et R₂ représente un atome d'hydrogène, un groupement méthyle, éthyle ou tertiobutyle, un groupement méthoxy ou éthoxy.

A titre d'exemple non limitatifs on peut citer le phénol, l'anisole, l'orthocrésol, le métacrésol, le paracrésol, le tertiobutyl-4 phénol, le méthoxy-2 phénol, le méthoxy-4 phénol.

Le procédé selon l'invention s'applique particulièrement bien au phénol pour la préparation d'hydroquinone et de pyrocatéchine.

Le peroxyde d'hydrogène peut être utilisé sous la forme d'une solution aqueuse ayant généralement une concentration en peroxyde d'hydrogène supérieure à 20 % en poids. Le peroxyde d'hydrogène peut également être utilisé sous la forme d'une solution dans un solvant organique. Comme solvants organiques utilisables pour la mise en oeuvre du peroxyde d'hydrogène, on peut utiliser des esters tels que notamment les esters d'alkyle ou de cycloalkyle d'acides carboxyliques aliphatiques saturés ; de préférence on emploiera les acétates et les propionates d'alkyle ayant au total de 4 à 8 atomes de carbone ou des mélanges de tels esters. On peut également utiliser des solutions de peroxyde d'hydrogène dans un éther tel que par exemple le dioxanne, le diisopropyléther ou le méthyl- tertiobutyléther.

Le rapport molaire composé de formule I/peroxyde d'hydrogène est généralement de 25/1 à 3/1 et préférentiellement de 20/1 à 4/1.

La quantité d'argile acide définie précédemment, que l'on peut mettre en oeuvre dans le présent procédé peut varier dans de très larges limites.

Lorsque l'on réalise le procédé en discontinu, le catalyseur peut représenter en poids par rapport au poids du composé de formule (I) engagé de 0,1 % à 20 %. De préférence, ce rapport pondéral sera compris entre 0,5 % et 10 %. Cependant si l'on réalise le procédé en continu, par exemple en faisant réagir un mélange de composé (I) et de solution de peroxyde d'hydrogène sur un lit fixe de catalyseur, ces rapports catalyseur/composé (I) n'ont plus de sens et, à un instant donné, on pourra avoir un excès pondéral de catalyseur par rapport au composé (I).

Il est également possible d'effectuer la réaction d'hydroxylation du composé (I) dans un solvant du composé (I), qui soit de préférence miscible ou partiellement miscible à l'eau.

A titre d'exemples non limitatifs de tels solvants, on peut citer l'eau ; les alcools comme le méthanol, le tertiobutanol, l'isopropanol ou l'éthanol ; les cétones comme l'acétone ou la méthylisobutylcétone ; les nitriles comme l'acétonitrile ; les acides carboxyliques comme l'acide acétique ; les esters comme l'acétate de propyle ; les éthers comme le méthyl-tertiobutyléther ; des solvants aprotiques polaires comme le dioxyde de tétrahydrothiophène (sulfolane), le carbonate d'éthylène glycol, le carbonate de propylène glycol, la N-méthylpyrrolidone.

La température à laquelle est conduite la réaction est généralement comprise entre 45° et 160°C sous pression atmosphérique. On peut également opérer à plus haute température et sous pression supérieure à la pression atmosphérique.

Les exemples qui suivent illustrent l'invention.

### EXEMPLE 1

Dans un réacteur en verre pyrex de 30 cm³, muni d'une agitation centrale par barreau aimanté, d'un réfrigérant relié à un gazomètre, d'un système de chauffage régulé et d'un système d'injection, on charge après avoir préalablement purgé l'appareil à l'aide d'azote :
- 9,4 g de phénol (0,1 mole)
- 0,25 g d'argile de type montmorillonite à propriétés acides
   (pH mesuré dans les conditions décrites : 2,75)
   (réf. KSF de SUD-CHEMIE, Munich)

On chauffe à 80°C sous agitation, puis on injecte une solution aqueuse de H₂O₂ à 70 % en poids par volume. (0,005 mole de H₂O₂).

On laisse ensuite réagir pendant 2 heures 30 minutes.

Après filtration du catalyseur, on dose H₂O₂ non transformée par iodométrie et les diphénols par chromatographie liquide haute performance (CLHP).

On obtient les résultats suivants :

| | |
|---|---|
| - taux de transformation (TT)de H₂O₂ : | 85,0 % |
| - rendement en pyrocatéchine par rapport à H₂O₂ transformée (RT) : | 30,0 % |
| - rendement en hydroquinone par rapport à H₂O₂ transformée (RT) : | 11,5 % |
| - rendement total en diphénols : | 41,5 % |

### EXEMPLE 2

On répète l'exemple 1 avec les mêmes quantités de réactifs, mais l'essai est réalisé à 130°C au lieu de 80°C.

On obtient les résultats suivants :

| | |
|---|---|
| - taux de transformation (TT)de H₂O₂ : | 99,0 % |
| - rendement en pyrocatéchine par rapport à H₂O₂ transformée (RT) : | 12,0 % |
| - rendement en hydroquinone par rapport à H₂O₂ transformée (RT) : | 6,0 % |
| - rendement total en diphénols : | 18,0 % |

### EXEMPLES 3 à 11

Dans un réacteur en verre pyrex de 30 cm3, muni d'une agitation magnétique, d'un réfrigérant relié à un gazomètre, d'un système de chauffage régulé et d'un système d'injection, on charge après avoir préalablement purgé l'appareil à l'aide d'azote :
- 4,7 g de phénol
- 0,25 g d'argile acide (KSF) utilisée dans l'exemple 1
- 4,7 g d'un solvant organique (voir tableau ci-après).

On chauffe à 80°C sous agitation, puis on injecte 0,125 g d'une solution de H₂O₂ à 70 % en poids par volume (2,5 mmol).

On laisse ensuite réagir pendant 2 h 30 minutes.

Après filtration du catalyseur, on dose H₂O₂ non transformée par iodométrie et les diphénols par chromatographie liquide haute performance (CLHP).

Le tableau ci-après rassemble les résultats obtenus (pyrocatéchine = PC ; hydroquinone = HQ).

| EXEMPLES | SOLVANTS | TT % H₂O₂ | RT % HQ | RT % PC | Somme RT % |
|---|---|---|---|---|---|
| Ex. 3 | acide acétique | 92,5 | 7,0 | 6,5 | 13,5 |
| Ex. 4 | acétonitrile | 84,0 | 4,5 | 8,5 | 13,0 |
| Ex. 5 | méthyl-isobutylcétone | 85,5 | 8,5 | 18,0 | 26,5 |
| Ex. 6 | acétate d'isopropyle | 93,5 | 1,0 | 3,5 | 4,5 |
| Ex. 7 | tertiobutanol | 29,0 | 1,0 | 6,5 | 7,5 |
| Ex. 8 | sulfolane | 91,5 | 5,0 | 7,5 | 12,5 |
| Ex. 9 | méthyl-tertiobutyléther | 88,0 | 1,5 | 3,0 | 4,5 |
| Ex. 10 | méthanol | 93,0 | 6,0 | 9,0 | 15,0 |
| Ex. 11 | eau | 94,0 | 23,5 | 39,0 | 62,5 |

### EXEMPLE 12

Dans un réacteur en verre pyrex de 30 cm³, muni d'une agitation centrale par barreau aimanté, d'un réfrigérant relié à un gazomètre, d'un système de chauffage régulé et d'un système d'injection, on charge après avoir préalablement purgé l'appareil à l'aide d'azote :
- 9,4 g de phénol (0,1 mole)
- 0,25 g d'argile de type montmorillonite à propriétés acides
   (pH mesuré dans les conditions décrites : 4,0)
   (réf. TONSIL OPTIMUM FF)

On chauffe à 80°C sous agitation, puis on injecte une solution aqueuse de H₂O₂ à 70 % en poids par volume. (0,005 mole de H₂O₂).

On laisse ensuite réagir pendant 2 heures 30 minutes.

Après filtration du catalyseur, on dose H₂O₂ non transformée par iodométrie et les diphénols par chromatographie liquide haute performance (CLHP).

On obtient les résultats suivants :

| | |
|---|---|
| - taux de transformation (TT)de H₂O₂ : | 97,0 % |
| - rendement en pyrocatéchine par rapport à H₂O₂ transformée (RT) : | 14,0 % |
| - rendement en hydroquinone par rapport à H₂O₂ transformée (RT) : | 9,0 % |
| - rendement total en diphénols : | 23,0 % |

### EXEMPLE 13

On répète l'exemple 12 avec les mêmes quantités de réactifs, mais l'essai est réalisé à 130°C au lieu de 80°C.

On obtient les résultats suivants :

| | |
|---|---|
| - taux de transformation (TT)de H₂O₂ : | 99,5 % |
| - rendement en pyrocatéchine par rapport à H₂O₂ transformée (RT) : | 11,5 % |
| - rendement en hydroquinone par rapport à H₂O₂ transformée (RT) : | 9,5 % |
| - rendement total en diphénols : | 21,0 % |

### EXEMPLE 14

On répète l'exemple 1 dans les mêmes conditions opératoires et avec les mêmes charges de réactifs, mais en utilisant comme catalyseur une argile de type VOLCLAY lavée pendant 2 heures à reflux à l'aide d'une solution aqueuse à 10 % d'acide chlorhydrique.
(pH mesuré dans les conditions décrites : 3,0)
Le traitement et les dosages sont les mêmes que pour l'exemple 1.

On obtient les résultats suivants :

| | |
|---|---|
| - taux de transformation de H₂O₂ : | 99,0 % |
| - rendement en pyrocatéchine par rapport à H₂O₂ transformée : | 12,0 % |
| - rendement en hydroquinone par rapport à H₂O₂ transformée : | 7,5 % |
| - rendement en diphénols : | 19,5 % |

### EXEMPLE 15

On répète l'exemple 1 dans les mêmes conditions opératoires et avec les mêmes charges de réactifs, mais en utilisant comme catalyseur une argile de type VOLCLAY lavée pendant 2 heures à reflux à l'aide d'une solution aqueuse à 10 % d'acide sulfurique.
(pH mesuré dans les conditions décrites : 3,0)
Le traitement et les dosages sont les mêmes que pour l'exemple 1.

On obtient les résultats suivants :

| | |
|---|---|
| - taux de transformation de H₂O₂ : | 99,0 % |
| - rendement en pyrocatéchine par rapport à H₂O₂ transformée : | 13,0 % |
| - rendement en hydroquinone par rapport à H₂O₂ transformée : | 7,5 % |
| - rendement en diphénols : | 20,5 % |

### EXEMPLE 16

On répète l'exemple 12 dans les mêmes conditions opératoires et avec les mêmes charges de réactifs, mais en chargeant avec le catalyseur de l'acide phosphorique (4 % en poids par rapport à H₂O₂).

Le traitement et les dosages sont les mêmes que pour l'exemple 12.

On obtient les résultats suivants :

| | |
|---|---|
| - taux de transformation de H₂O₂ | 98,5 % |
| - rendement en pyrocatéchine par rapport à H₂O₂ transformée | 19,0 % |
| - rendement en hydroquinone par rapport à H₂O₂ transformée | 12,5 % |
| - rendement en diphénols | 31,5 % |

### EXEMPLE 17

On répète l'exemple 1 dans les mêmes conditions opératoires et avec les mêmes charges de réactifs, mais en utilisant comme catalyseur une argile de type VOLCLAY modifiée par un traitement avec de l'argile chlorhydrique dilué.
(pH mesuré dans les conditions décrites : 3,5)
Le traitement et les dosages sont les mêmes que pour l'exemple 1.

On obtient les résultats suivants :

| | |
|---|---|
| - taux de transformation de H₂O₂ | 57,0 % |
| - rendement en pyrocatéchine par rapport à H₂O₂ transformée | 30,0 % |
| - rendement en hydroquinone par rapport à H₂O₂ transformée | 15,0 % |
| - rendement en diphénols | 45,0 % |

### EXEMPLE 18

On répète l'exemple 1 dans les mêmes conditions opératoires et avec les mêmes charges de réactifs, mais en utilisant comme catalyseur une argile de type VOLCLAY modifiée par un traitement à l'acide phosphorique dilué.
(pH mesuré dans les conditions décrites : 3,5)
Le traitement et les dosages sont les mêmes que pour l'exemple 1.

On obtient les résultats suivants :

| | |
|---|---|
| - taux de transformation de H₂O₂ | 84,0 % |
| - rendement en pyrocatéchine par rapport à H₂O₂ transformée | 21,0 % |
| - rendement en hydroquinone par rapport à H₂O₂ transformée | 11,0 % |
| - rendement en diphénols | 32,0 % |

A titre de comparaison un essai réalisé dans les mêmes conditions avec l'argile VOLCLAY brute (non acide : pH = 8,5) a conduit aux résultats suivants :

| | |
|---|---|
| - taux de transformation de H₂O₂ | 67,0 % |
| - rendement en pyrocatéchine par rapport à H₂O₂ transformée | 2,0 % |
| - rendement en hydroquinone par rapport à H₂O₂ transformée | 0,5 % |
| - rendement en diphénols | 2,5 % |

## Revendications

1. Procédé d'hydroxylation de phénols ou éthers de phénols de formule générale (I) : 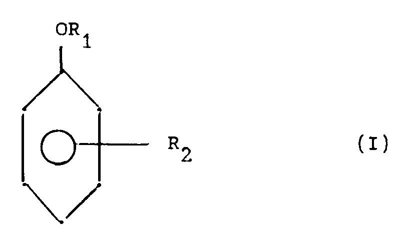 dans laquelle :
- R₁ représente un atome d'hydrogène, un groupement méthyle, un groupement éthyle ou un groupement phényle,
- R₂ représente un atome d'hydrogène, un radical alkyle ayant 1 à 4 atomes de carbone, un radical alkoxy ayant 1 à 4 atomes de carbone, un radical phényle ou cyclohexyle ;
par réaction avec le peroxyde d'hydrogène, caractérisé en ce que la réaction est conduite en présence d'une quantité efficace d'un catalyseur constitué d'au moins une argile acide.

2. Procédé selon la revendication 1, caractérisé en ce qu'il est appliqué aux phénols et éthers de phénol de formule (I) dans laquelle R₁ représente un atome d'hydrogène, un groupement méthyle ou un groupement éthyle, et R₂ représente un atome d'hydrogène, un groupement méthyle, éthyle ou tertiobutyle, un groupement méthoxy ou éthoxy.

3. Procédé selon l'une des revendications 1 ou 2 caractérisé en ce qu'il est appliqué aux phénols et éthers de phénols choisi parmi le phénol, l'anisole, l'orthocrésol, le métacrésol, le paracrésol, le tertiobutyl-4 phénol, le méthoxy-2 phénol, le méthoxy-4 phénol.

4. Procédé selon l'une des revendications 1 à 3, caractérisé en ce que l'argile acide présente, en suspension dans l'eau, un pH compris entre 2 et 6.

5. Procédé selon l'une des revendications 1 à 4, caractérisé en ce que l'argile acide utilisée est choisie parmi les argiles naturelles présentant une structure dite "TOT" ou tetraèdre-octaèdre-tétraèdre.

6. Procédé selon l'une des revendications 1 à 5, caractérisé en ce que l'argile acide utilisée est choisie parmi les smectites, les vermiculites, et les micas et de préférence parmi les smectites.

7. Procédé selon la revendication 6, caractérisé en ce que l'argile acide utilisée est choisie parmi :
- les montmorillonites de formule générale Si₄(Al_{2-y}-Mg_{y})O₁₀OH₂,M⁺_{y}
- les beidellites de formule (Si₄₋ₓAlₓ)Al₂O₁₀OH₂, M⁺ₓ
- les nontronites de formule (Si₄₋ₓAlₓ)Fe₂O₁₀OH₂,M⁺ₓ
- les hectorites de formule Si₄(Mg_{3-y}-Li_{y})O₁₀OH₂,M⁺_{y}
- les stévensites de formule Si₄(Mg_{3-y})O₁₀OH₂,M⁺_{2y}
- les saponites de formule (Si₄₋ₓAlₓ)Mg₃O₁₀OH₂,M⁺ₓ.
et de préférence les montmorillonites.

8. Procédé selon l'une des revendications 1 ou 7, caractérisé en ce que l'on traite les argiles commerciales, déjà acides ou non, par une solution aqueuse d'un acide.

9. Procédé selon la revendication 8, caractérisé en ce que la concentration de la solution aqueuse en acide est telle que son pH soit supérieur ou égal à 2 et et qu'elle contient une quantité d'ion H⁺ exprimé en milliéquivalents correspondant à au moins la capacité d'échange de l'argile.

10. Procédé selon la revendication 9, caractérisé en ce que la concentration de la solution aqueuse en acide est telle qu'elle contient une quantité d'ion H⁺ d'au moins 0,6 et de préférence d'au moins 1 milliéquivalent acide pour 100 g d'argile.

11. Procédé selon l'une des revendications 8 à 18, caractérisé en ce que le traitement acide est effectué à l'aide d'acide chlorhydrique, d'acide sulfurique, d'acide nitrique, d'acide trifluorométhanesulfonique ou d'acide phosphorique.

12. Procédé selon l'une des revendications 8 à 10, caractérisé en ce que le traitement acide est suivi d'un traitement par un alcool.

13. Procédé selon l'une des revendications 1 à 12 réalisé en discontinu, caractérisé en ce que le catalyseur représente en poids par rapport au poids du composé de formule (I) engagé de 0,1 % à 20 % et de préférence de 0,5 % à 10 %.

14. Procédé selon l'une des revendications 1 à 12, caractérisé en ce que le procédé est réalisé en continu sur un lit fixe de catalyseur.

15. Procédé selon l'une des revendications 1 à 14, caractérisé en ce que le peroxyde d'hydrogène est mis en oeuvre sous la forme d'une solution aqueuse.

16. Procédé selon l'une des revendications 1 à 15, caractérisé en ce que le peroxyde d'hydrogène est mis en oeuvre sous la forme d'une solution organique.

17. Procédé selon l'une des revendications 1 à 16, caractérisé en ce que la réaction d'hydroxylation est effectuée dans un solvant du composé de formule (I), de préférence miscible ou partiellement miscible à l'eau, tel que l'eau, un alcool, une cétone, un nitrile, un acide carboxylique, un ester, un éther ou un solvant aprotique polaire.

18. Procédé selon l'une des revendications 1 à 17, caractérisé en ce que la réaction d'hydroxylation est conduite à une température comprise entre 45°C et 160°C.

## Claims

1. Process for the hydroxylation of phenols or ethers of phenols of general formula (I): 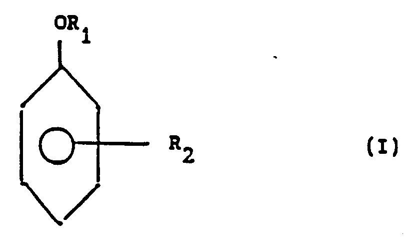 in which:
- R₁ represents a hydrogen atom, a methyl group, an ethyl group or a phenyl group,
- R₂ represents a hydrogen atom, an alkyl radical having 1 to 4 carbon atoms, an alkoxy radical having 1 to 4 carbon atoms or a phenyl or cyclohexyl radical;
by reaction with hydrogen peroxide, characterized in that the reaction is performed in the presence of an effective quantity of a catalyst constituted by at least one acid clay.

2. Process according to claim 1, characterized in that it is applied to phenols and phenol ethers of formula (I), in which R₁ represents a hydrogen atom, a methyl group or an ethyl group and R₂ represents a hydrogen atom, a methyl, ethyl, tert.butyl, methoxy or ethoxy group.

3. Process according to one of the claims 1 or 2, characterized in that it is applied to phenols and phenol ethers chosen from among phenol, anisole, orthocresol, metacresol, paracresol, 4-tert.butyl phenol, 2-methoxy phenol and 4-methoxy phenol.

4. Process according to one of the claims 1 to 3, characterized in that the acid clay has, suspended in water, a pH between 2 and 6.

5. Process according to one of the claims 1 to 4, characterized in that the acid clay used is chosen from among natural clays having a so-called TOT or tetrahedral - octahedral - tetrahedral structure.

6. Process according to one of the claims 1 to 5, characterized in that the acid clay used is chosen from among smectites, vermiculites, micas and preferably from among smectites.

7. Process according to claim 6, characterized in that the acid clay used is chosen from among:
- montmorillonites of general formula: Si₄(Al_{2-y}-Mg_{y})O₁₀OH₂,M⁺_{y}
beidellites of formula (Si₄₋ₓAlₓ)Al₂O₁₀OH₂,M⁺ₓ
nontronites of formula (Si₄₋ₓAlₓ)Fe₂O₁₀OH₂,M⁺ₓ
hectorites of formula Si₄(Mg_{3-y}-Li_{y})O₁₀OH₂,M⁺_{y}
stevensites of formula Si₄(Mg_{3-y})O₁₀OH₂,M⁺_{2y}
saponites of formula (Si₄₋ₓAlₓ)Mg₃O₁₀OH₂,M⁺ₓ
and preferably montmorillonites.

8. Process according to one of the claims 1 or 7, characterized in that the commercial clays, which may or may not be already acid, are treated by an aqueous solution of an acid.

9. Process according to claim 8, characterized in that the acid concentration of the aqueous solution is such that its pH is equal to or higher than 2 and it contains a H⁺ ion quantity, expressed in milliequivalents, corresponding to at least the exchange capacity of the clay.

10. Process according to claim 9, characterized in that the acid concentration of the aqueous solution is such that it contains an H⁺ ion quantity of at least 0.6 and preferably at least 1 acid milliequivalent per 100 g of clay.

11. Process according to one of the claims 8 to 18, characterized in that the acid treatment is carried out with the aid of hydrochloric, sulphuric, nitric, trifluoromethane sulphonic or phosphoric acid.

12. Process according to one of the claims 8 to 10, characterized in that the acid treatment is followed by a treatment by an alcohol.

13. Process according to one of the claims 1 to 12 performed discontinuously, characterized in that the catalyst represents, by weight, based on the weight of the compound of formula (I) used, 0.1 to 20% and preferably 0.5 to 10%.

14. Process according to one of the claims 1 to 12, characterized in that the process is performed continuously on a fixed catalyst bed.

15. Process according to one of the claims 1 to 14, characterized in that the hydrogen peroxide is performed in the form of an aqueous solution.

16. Process according to one of the claims 1 to 15, characterized in that the hydrogen peroxide is performed in the form of an organic solution.

17. Process according to one of the claims 1 to 16, characterized in that the hydroxylation reaction is performed in a solvent of the compound of formula (I), which is preferably miscible or partly miscible with water, such as water, an alcohol, a ketone, a nitrile, a carboxylic acid, an ester, an ether or an aprotic polar solvent.

18. Process according to one of the claims 1 to 17, characterized in that the hydroxylation reaction is performed at a temperature between 45 and 160°C.

## Patentansprüche

1. Verfahren zur Hydroxylierung von Phenolen oder Phenolethern der allgemeinen Formel (I): 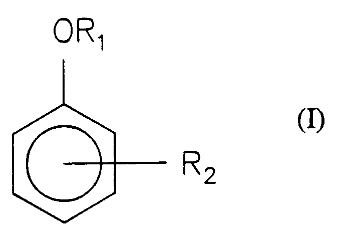 in der:
- R₁ ein Wasserstoffatom, eine Methylgruppe, eine Ethylgruppe oder eine Phenylgruppe darstellt,
- R₂ ein Wasserstoffatom, einen Alkylrest mit 1 bis 4 Kohlenstoffatomen, einen Alkoxyrest mit 1 bis 4 Kohlenstoffatomen oder einen Phenyl- oder Cyclohexylrest darstellt;
durch Reaktion mit Wasserstoffperoxid, dadurch gekennzeichnet, daß die Reaktion in Gegenwart einer wirksamen Menge eines Katalysators durchgeführt wird, der zumindest aus einer sauren Tonerde besteht.

2. Verfahren nach Anspruch 1, dadurch gekennzeichnet, daß es auf Phenole und Phenolether der Formel (I) angewendet wird, in der R₁ ein Wasserstoffatom, eine Methylgruppe oder eine Ethylgruppe darstellt und R₂ ein Wasserstoffatom, eine Methyl-, Ethyl- oder tert.-Butylgruppe oder eine Methoxy- oder Ethoxygruppe darstellt.

3. Verfahren nach einem der Ansprüche 1 oder 2, dadurch gekennzeichnet, daß es auf folgende Phenole und Phenolether angewendet wird: Phenol, Anisol, o-Kresol, m-Kresol, p-Kresol, 4-tert.-Butyl-phenol, 2-Methoxy-phenol, 4-Methoxy-phenol.

4. Verfahren nach einem der Ansprüche 1 bis 3, dadurch gekennzeichnet, daß die wässrige Suspension der sauren Tonerde einen pH-Wert zwischen 2 und 6 besitzt.

5. Verfahren nach einem der Ansprüche 1 bis 4, dadurch gekennzeichnet, daß die verwendete saure Tonerde eine natürliche Tonerde ist, die eine sogenannte "TOT"- oder Tetraeder-Oktaeder-Tetraeder-Struktur besitzt.

6. Verfahren nach einem der Ansprüche 1 bis 5, dadurch gekennzeichnet, daß die verwendete saure Tonerde aus den Smectiten, den Vermiculiten und den Glimmern ausgewählt wird, wobei vorzugsweise Smectiten verwendet werden.

7. Verfahren nach Anspruch 6, dadurch gekennzeichnet, daß die verwendete saure Tonerde unter folgenden ausgewählt wird:
- den Montmorilloniten der allgemeinen Formel: Si₄(Al_{2-y}Mg_{y})O₁₀OH₂, M⁺_{y}
- den Beidelliten der Formel: (Si₄₋ₓAlₓ)Al₂O₁₀OH₂, M⁺ₓ
- den Nontroniten der Formel: (Si₄₋ₓAlₓ)Fe₂O₁₀OH₂, M⁺ₓ
- den Hectoriten der Formel: Si₄(Mg_{3-y}Li_{y})O₁₀OH₂, M⁺_{y}
- den Stevensiten der Formel: Si₄(Mg_{3-y})O₁₀OH₂, M⁺_{2y}
- den Saponiten der Formel: (Si₄₋ₓAlₓ)Mg₃O₁₀OH₂, M⁺ₓ
wobei vorzugsweise die Montmorillonite verwendet werden.

8. Verfahren nach einem der Ansprüche 1 oder 7, dadurch gekennzeichnet, daß die kommerziell erhältlichen Tone, bereits azide oder nicht, mit einer wässrigen Säurelösung behandelt werden.

9. Verfahren nach Anspruch 8, dadurch gekennzeichnet, daß die wässrige Säurelösung in einer solchen Konzentration vorliegt, daß der pH-Wert dieser Lösung größer oder gleich 2 ist, und daß diese Lösung mindestens soviele Milliäquivalente Protonen enthält, wie der Austauschkapazität der Tonerde entspricht.

10. Verfahren nach Anspruch 9, dadurch gekennzeichnet, daß die wässrige Säurelösung in einer solchen Konzentration vorliegt, daß sie mindestens 0,6, vorzugsweise mindestens 1 Milliäquivalent Protonen auf 100 g Tonerde enthält.

11. Verfahren nach einem der Ansprüche 8 bis 18, dadurch gekennzeichnet, daß die Säurebehandlung mit Hilfe von Salzsäure, Schwefelsäure, Salpetersäure, Trifluormethansulfonsäure oder von Phosphorsäure durchgeführt wird.

12. Verfahren nach einem der Ansprüche 8 bis 10, dadurch gekennzeichnet, daß der Säurebehandlung eine Alkoholbehandlung folgt.

13. Verfahren nach einem der Ansprüche 1 bis 12, das diskontinuierlich durchgeführt wird, dadurch gekennzeichnet, daß das Gewicht des Katalysators, bezogen auf das Gewicht der eingesetzten Verbindung der Formel (I), 0,1 % bis 20 %, vorzugsweise 0,5 % bis 10%, beträgt.

14. Verfahren nach einem der Ansprüche 1 bis 12, dadurch gekennzeichnet, daß das Verfahren auf einem festen Katalysatorbett kontinuierlich durchgeführt wird.

15. Verfahren nach einem der Ansprüche 1 bis 14, dadurch gekennzeichnet, daß das Wasserstoffperoxid in Form einer wässrigen Lösung eingesetzt wird.

16. Verfahren nach einem der Ansprüche 1 bis 15, dadurch gekennzeichnet, daß das Wasserstoffperoxid in Form einer organischen Lösung eingesetzt wird.

17. Verfahren nach einem der Ansprüche 1 bis 16, dadurch gekennzeichnet, daß die Hydroxylierungsreaktion in einem Lösungsmittel durchgeführt wird, das die Verbindung der Formel (I) löst und das vorzugsweise ganz oder teilweise mit Wasser mischbar ist, wie zum Beispiel Wasser, ein Alkohol, ein Keton, ein Nitril, eine Carbonsäure, ein Ester, ein Ether oder ein polares, aprotisches Lösungsmittel.

18. Verfahren nach einem der Ansprüche 1 bis 17, dadurch gekennzeichnet, daß die Hydroxylierungsreaktion bei einer Temperatur zwischen 45°C und 160°C durchgeführt wird.
